Europäisches Patentamt

European Patent Office

Office européen des brevets

(11) Publication number: **0 338 994**
**A1**

(12) **EUROPEAN PATENT APPLICATION**

(21) Application number: **89830006.6**

(22) Date of filing: **10.01.89**

(51) Int. Cl.4: **A 61 F 2/24**
**A 61 B 19/00**

(30) Priority: **12.01.88 IT 6701488**

(43) Date of publication of application:
**25.10.89 Bulletin 89/43**

(84) Designated Contracting States:
**AT BE CH DE ES FR GB GR IT LI LU NL SE**

(71) Applicant: **Morea, Mario**
**Via San Quintino 10**
**I-10121 Torino (IT)**

**De Paulis, Ruggero**
**Strada Moncalvo 50,**
**I-10024 Moncalieri (Torino) (IT)**

(72) Inventor: **Morea, Mario**
**Via San Quintino 10**
**I-10121 Torino (IT)**

**De Paulis, Ruggero**
**Strada Moncalvo 50,**
**I-10024 Moncalieri (Torino) (IT)**

(74) Representative: **Bosotti, Luciano et al**
**c/o Jacobacci-Casetta & Perani S.p.A. Via Alfieri, 17**
**I-10121 Torino (IT)**

(54) Prosthetic device for the surgical correction of tricuspid insufficiency.

(57) The device (1) comprises a shaped and flexible segment constituted by a supporting core and a fabric covering applied to the supporting core, and can be sutured to the ring of the tricuspid valve at least in the section between the antero-septal and the postero-septal commissures. The segment (1) extends along an elliptical line having dimensions (X,Y) and an orientation (α) which are determined relative to the chord (C) defined by the two ends of the segment (4, 5) which are intended to be sutured to the commissures. On its inner side, the device has a lip formation (6) through which suture stitches can be passed to prevent the stitches from sliding along the device. To advantage, the latter may be incorporated in a surgical kit including lengths of suture thread with respective surgical needles already attached so that it can be fitted more quickly.

1/2

FIG. 1

EP 0 338 994 A1

## Description

### Prosthetic device for the surgical correction of tricuspid insufficiency

#### Background of the invention

The present invention relates to prostheses for the surgical correction of tricuspid insufficiency.

Such a heart condition is generally found to be due to an anomalous dilation of the ring or annulus of the tricuspid valve which occurs particularly in correspondence with the antero-lateral flap and the poster-lateral flap of the valve itself.

This dilation means that it is not possible to achieve complete adhesion or fitting between the free edges of the valve flaps or leaflets in the systolic phase: in other words, the valve does not close completely, so that there is a reduction in the pumping action achieved by the heart muscle.

A surgical technique is known (De Vega) which enables tricuspid insufficiency to be corrected by reducing the expanded annulus with a simple suture until its cross-section is brought to a functionally correct value.

This technique is unsatisfactory, particularly since it is difficult precisely to control the reduction of the cross-section of the dilated annulus: in some cases, this technique may even lead to the formation of a valve stenosis.

In order to remedy this problem, it has been proposed in the past to use prostheses constituted by open or closed rings which are intended to be arranged in the plane of the annulus so as to bring the expanded annulus back to the dimensions of the prosthetic ring by means of a suture operation.

In particular, the use of a prosthesis comprising a shaped segment which can be sutured to the ring of the tricuspid valve has been proposed (Carpentier) for the surgical correction of tricuspid insufficiency. Although the results obtainable may be considered entirely satisfactory, the technique for the implantation of this device requires quite long operating times.

#### Object of the invention

The object of the present invention, therefore, is to produce a prosthesis of the type specified above which does not give rise to the problems described above.

#### Summary of the invention

According to the present invention, this object is achieved by virtue of a prosthesis for the surgical correction of tricuspid insufficiency, characterised in that it comprises a shaped segment which can be sutured to the ring (annulus) of the tricuspid valve in the section between the antero-septal and postero-septal commissures and opposite the septal flap, the segment being shaped to follow a substantially elliptical line with two ends which can be sutured to the commissures. Preferably, the two ends define a chord of the elliptical line and the ratio between the length of the chord and the length of the major axis of the ellipse is between about 0.6 and 0.8, preferably substantially 0.7.

According to the solution which is currently considered the best, the chord is at an angle α to the major axis of the ellipse of between about 17 and 23 degrees, preferably substantially 20 degrees. The ratio between the length of the major axis of the elliptical line and the length of its minor axis is between about 1.5 and 2.3, preferably 1.9.

It should be stated that the term "substantially elliptical line" as used in the present description and in the claims which follow is intended to extend in general to all lines in which a major axis or diameter and a minor axis or diameter, as well as a chordal segment identified by the ends of the shaped segment constituting the prosthesis, can be distinguished.

A second aspect of the present invention concerns a prosthesis for the surgical correction of tricuspid insufficiency comprising a shaped segment which can be sutured to the ring of the tricuspid valve, characterised in that it has a lip formation on its inner side, through which suture stiches can be passed, the lip formation being substantially fixed against sliding longitudinally of the device. The device preferably includes a flexible, shaped supporting core and a fabric covering applied to the supporting core. In the preferred embodiment, both the supporting core and the covering fabric, a fold of which forms the lip formation, are made from biocompatible polymers such as, for example, the materials known under the trade names of Delrin and Dacron for the core and the covering respectively.

The device is preferably provided, at least on the fabric surrounding the core, with a thin covering of biocompatible carbon material applied by sputtering.

To advantage, the device according to the invention may be made up into a surgical kit including respective lengths of suture thread stitched to the device adjacent the two ends thereof, each length of thread carrying at least one associated surgical needle.

The presence of these lengths of suture thread already attached to the prosthesis enables a valuable saving in time at the implantation stage, making the operation quicker.

#### Detailed description of the invention

The invention will now be described, purely by way of non-limiting example, with reference to the appended drawings, in which :

Figure 1 is an anatomical drawing showing the position in which the device according to the invention is implanted,

Figure 2 is a plan view of the device according to the invention, from which the most important geometrical parameters of the device can be seen,

Figure 3 is a section taken on the line III-III of Figure 2, and

Figure 4 is a perspective view of the device of Figure 1 made up into an operating kit including suture threads and needles already partially attached to the device.

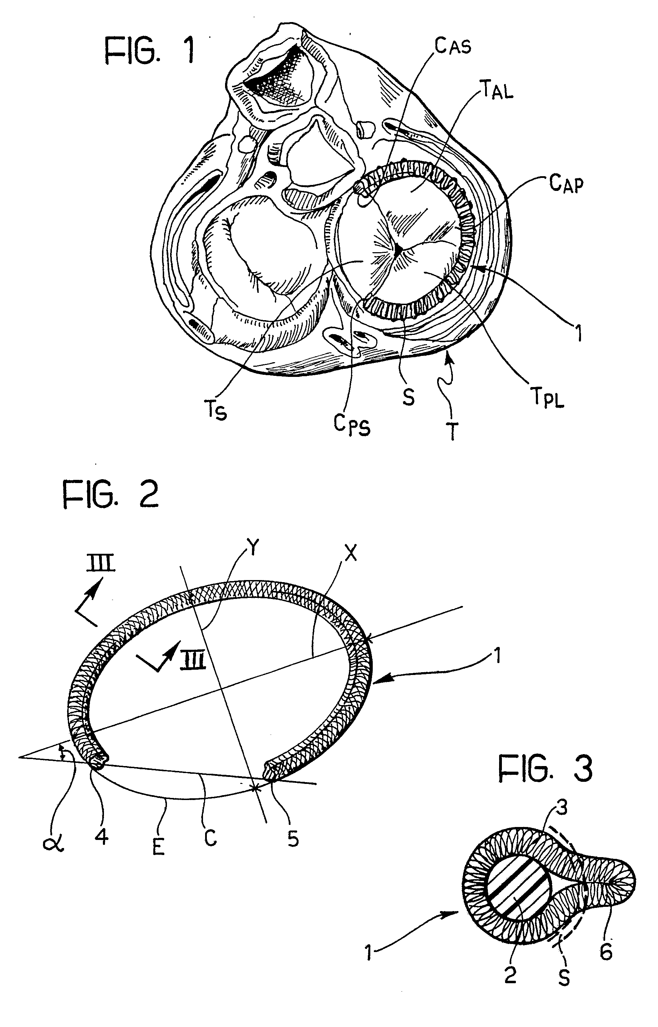

Figure 1 shows schematically a heart with the atria removed, seen from below in the systolic phase.

In the heart illustrated, the tricuspid valve T can be seen, with its three flaps or leaflets constituted respectively by:
- a septal flap $T_S$,
- an antero-lateral flap $T_{AL}$, and
- a postero-septal flap $T_{PL}$.

The flaps $T_S$, $T_{AL}$ and $T_{PL}$ extend from the so-called valve ring or annulus and are connected to each other in correspondence with the so-called commissures, constituted more precisely by:
- an antero-septal commissure $C_{AS}$,
- a postero-septal commissure $C_{PS}$, and
- an antero-posterior commissure $C_{AP}$.

The prosthesis according to the invention, generally indicated 1, is intended to be fitted in the plane of the annulus of the tricuspid valve substantially in the section which corresponds to the antero-lateral flap $T_{AL}$ and the postero-lateral flap $T_{PL}$. In fact, it is in correspondence with these two flaps that the dilation which gives rise to tricuspid insufficiency mainly occurs.

In other words, in the terminology used in the following claims, the device 1 according to the invention is intended to be sutured to the ring of the tricuspid valve in the section between the antero-septal commissure ($C_{AS}$) and the postero-septal commissure ($C_{PS}$) and opposite the septal flap ($T_S$).

In fact, it is possible to bring the annulus which is dilated as a result of the heart condition back to its functionally-correct shape by the application of suture stitches S.

Naturally, this shape is not the same for the whole population, particularly as regards its dimensions. As in the case of other heart prostheses (for example, mechanical or biological valve prostheses) it is possible to identify a certain number of dimensional classes in the patient population so that the surgeon can be provided with an assortment of prostheses of different dimensions. The surgeon then selects from the assortment the prosthesis best suited to the operative situation, during the operation, on the basis of dimensional measurements made at the time (for example, by means of plugs).

As can better be seen in the plan view of Figure 2, the device 1 according to the invention is characterised in that it extends along a substantially elliptical line E (as defined above) in which a major axis X and a minor axis Y can be distinguished.

More precisely, as will be explained more fully below, the device 1 is constituted by a shaped and flexible core 2 surrounded by a covering 3 which is preferably constituted by a fabric of a biocompatible-polymer thread.

The descriptions shaped and flexible, used in references to the core 2 and to the device 1 as a whole in the present description and in the following claims, are intended to indicate that, whilst these items are resiliently deformable so as to adapt better to their implanted situation (tissues which deform with the beating of the heart), the core 2 and the device 1 as a whole have their own shapes to which they return in the absence of external stresses and to which the annulus of the tricuspid valve sutured to the device tends to be restored. In particular, the core 2 and the device 1 as a whole are substantially inextensible longitudinally.

The device 1 has two ends 4 and 5 which are intended to be sutured to the antero-septal commissure $C_{AS}$ (end 4) and to the postero-septal commissure $C_{PS}$ (end 5) respectively.

With the line E, the two ends 4 and 5 define a chord C whose length is in a ratio of between about 0.6 and 0.8, preferably substantially 0.7, to the length of the major axis X of the line E.

The chord C is at an angle ($\alpha$) to the major axis X which may be between about 17 and 23 degrees, preferably substantially 20 degrees.

The overall shape of the line E is selected so that the ratio between the length of its major axis X and the length of its minor axis Y is within the range from about 1.5 to about 2.3, and is preferably substantially 1.9.

As can better be seen in the section of Figure 3, the device 1 includes a shaped and flexible core 2 made from a biocompatible polymer (for example, the material known by the trade name Delrin) to which a covering constituted by a fabric of a biocompatible-polymer thread (for example, a thread of the material known by the trade name Dacron) is applied - according to known techniques of the type used for the manufacture of the suture rings of some heart valve prostheses.

The embodiment illustrated relates to a core 2 with a circular cross-section. However, cores with different cross-sections, for example, rectangular or elliptical, may be used.

On the inner side of the element 1, that is, the side intended to face the valve opening, the covering 3 has a lip formation 6 through which the suture stitches sewn to the device 1 during the implanting operation (which are shown schematically by broken lines and indicated S in Figure 3) can easily pass.

The lip formation 6 is preferably constituted by a fold of the covering 3. The latter is wrapped sufficiently loosely around the core 2 and the fold 6 is then formed by means of a stitching, sewing or a shaping operation carried out by any known method, the covering being closed in correspondence with the ends 4 and 5.

The lip formation 6 is thus substantially fixed against longitudinal movement relative to the core 2 and the device 1 as a whole. In particular, this means (see Figure 1) that the suture stitches S do not tend to "slide" along the device 1. This ensures that the tissue of the annulus which is drawn in by means of suturing to the implant 1 retains exactly the configuration imparted to it during the operation and does not tend to be gathered towards the central region of the device 1 near the antero-posterior commissure.

The presence of the lip formation 6 facilitates the sewing of the suture stitches S. The device 1 as a whole, or at least the covering fabric 3, may be covered by a thin layer of biocompatible carbon material which can be deposited according to the criteria described in European patent applications Nos. 0,102,328 and 0,224,080.

Preferably, the device 1 is fitted by the suturing of the annulus starting from the ends 4 and 5 which are intended to be sutured to the antero-septal commissure $C_{AS}$ and the postero-septal commissure $C_{PS}$. It is therefore important to be able to fix the device firmly to the physiological tissue in these regions. Thus, it is preferable that the suture stitches in correspondence with these ends are firmly connected to the device 1 by means of U-shaped stitches which preferably extend through the core 2 of the device 1.

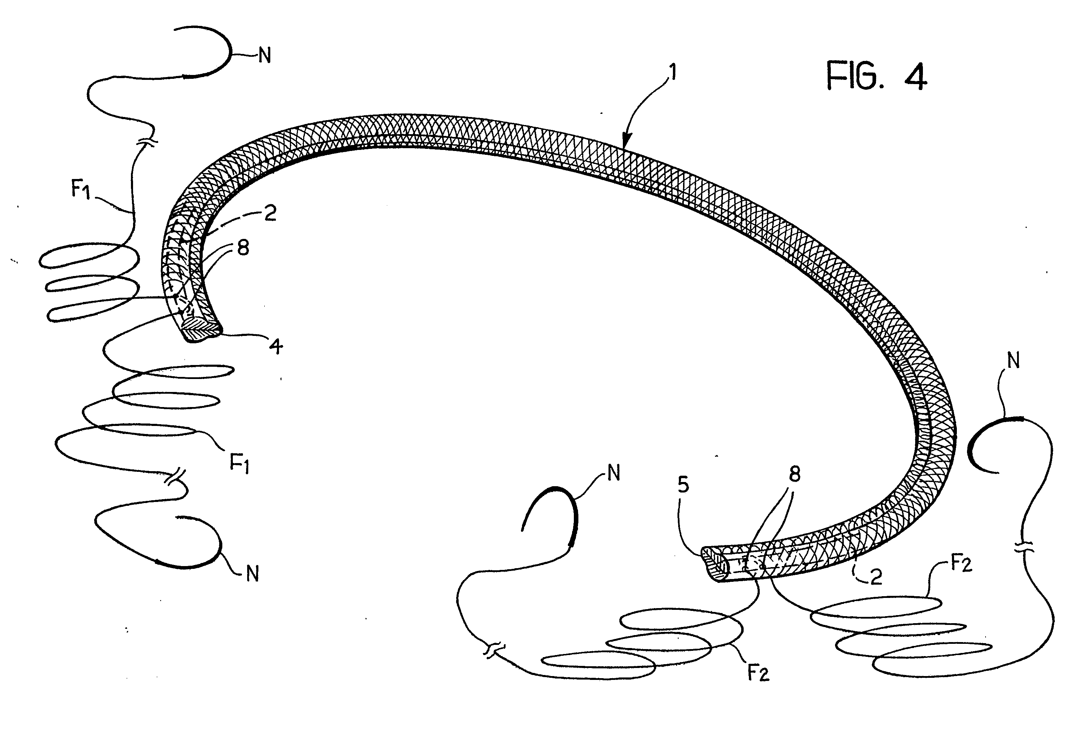

Since the formation of stitches of this type would involve a loss of time at the stage of the implanting operation, the device 1 according to the invention may usefully be made up into a surgical kit in which respective lengths $F_1$ and $F_2$ of surgical thread are already associated with the two ends 4 and 5 of the device and are fixed to the device 1, for example, by U-shaped stitches which pass through holes 8 provided in the core 2, as shown schematically in Figure 4.

Each length of thread $F_1$, $F_2$ is generally U-shaped and thus includes a loop part, which extends through one end of the device 1, and two lateral branches which are intended to be used to complete the suture operation.

For this purpose, both these two lateral branches to advantage carry respective surgical needles N already connected to their free ends.

Once the operating zone has been isolated and the device 1 located in the implant position, the surgeon can begin suturing immediately, minimising the time necessary to complete the operation.

## Claims

1. A prosthetic device for the surgical correction of tricuspid insufficiency, characterised in that it comprises a shaped segment which can be sutured to the ring of the tricuspid valve in the section between the antero-septal commissure ($C_{AS}$) and the postero-septal commissure ($C_{PS}$) and opposite the septal flap ($T_S$), the segment being shaped to follow a substantially elliptical line (E) with two ends (4, 5) which can be sutured to the commissures ($C_{AS}$, $C_{PS}$).

2. A device according to Claim 1, characterised in that the two ends (4,5) define a chord (C) of the elliptical line (E), the ratio between the length of the chord (C) and the major axis (X) of the elliptical line being between about 0.6 and 0.8.

3. A device according to Claim 2, characterised in that the ratio between the length of the chord (C) and the length of the major axis (X) of the elliptical line (E) is substantially 0.7.

4. A device according to any one of Claims 1 to 3, characterised in that the two ends (4, 5) define a chord (C) of the elliptical line (C) which is at an angle ($\alpha$) of between about 17 degrees and about 23 degrees to the major axis of elliptical line (E).

5. A device according to Claim 4, characterised in that the chord (5) is at an angle ($\alpha$) of substantially 20 degrees to the major axis (X) of the elliptical line (E).

6. A device according to any one of Claims 1 to 5, characterised in that the ratio between the length of the major axis (X) of the elliptical line (E) and the length of its minor axis (Y) is between about 1.5 and 2.3.

7. A device according to Claim 6, characterised in that the ratio between the length of the major axis (X) of the elliptical line and the length of its minor axis (Y) is substantially 1.9.

8. A prosthetic device for the surgical correction of tricuspid insufficiency, comprising a shaped segment which can be sutured to the ring of the tricuspid valve, characterised in that it has a lip formation (6) on its inner side through which suture stitches (S) can easily pass, the lip formation (6) being substantially fixed against sliding longitudinally of the device (1).

9. A device according to Claim 8, characterised in that it includes a shaped and flexible supporting core (2) and a fabric covering (3) applied to the supporting core (2).

10. A device according to Claim 9, characterised in that the lip formation (6) is constituted by a fold of the fabric covering (3).

11. A device according to any one of Claims 9 and 10, characterised in that the core (2) and the covering (3) are made from biocompatible polymers.

12. A device according to Claim 11, characterised in that a layer of biocompatible carbon material deposited by sputtering is provided, at least on the covering

13. A prosthetic device for the surgical correction of tricuspid insufficiency, comprising a shaped segment which can be sutured to the ring of the tricuspid valve and has at least two opposite ends (4, 5), characterised in that it includes, associated with the device in a surgical kit, respective lengths of suture thread ($F_1$, $F_2$) fixed to the device (1) adjacent the two ends (4, 5), each length of thread ($F_1$, $F_2$) carrying at least one associated surgical needle (N).

14. A device according to Claim 13, characterised in that a length of suture thread ($F_1$, $F_2$) is associated with each of the ends (4, 5) and extends along a generally U-shaped line with a loop part which passes through the device (1) and two branches, each of which carries an associated surgical needle (N).

15. A device according to Claim 9 and Claim 14, characterised in that the core (2) is provided with respective apertures (8) at each of the ends (4, 5), through which the loop part of a respective length of suture thread ($F_1$, $F_2$) passes.

FIG. 1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 164 046 (COOLEY) * Column 5, lines 8-45; figures 4,5; column 4, lines 5-10 * | 1,8-10 | A 61 F 2/24 A 61 B 19/00 |
| Y | | 11,12 | |
| Y | EP-A-0 102 328 (SORIN BIOMEDICA S.p.A.) * Abstract; page 5, lines 20-29 * | 11,12 | |
| D,A | US-A-3 656 185 (CARPENTIER) * Column 1, lines 30-39; figures 2,5,6 * | 1 | |
| A | US-A-4 042 979 (ANGELL) | | |

TECHNICAL FIELDS
SEARCHED (Int. Cl.4)

A 61 F
A 61 B

The present search report has been drawn up for all claims

| Place of search | Date of completion of the search | Examiner |
|---|---|---|
| THE HAGUE | 11-04-1989 | SANCHEZ Y SANCHEZ J. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)